(19) Europäisches Patentamt
European Patent Office
Office européen des brevets

(11) **EP 4 389 080 A1**

(12) **EUROPEAN PATENT APPLICATION**

(43) Date of publication:
**26.06.2024 Bulletin 2024/26**

(21) Application number: **23218009.1**

(22) Date of filing: **19.12.2023**

(51) International Patent Classification (IPC):
**A61F 2/38** (2006.01)     **A61F 2/30** (2006.01)

(52) Cooperative Patent Classification (CPC):
**A61F 2/384; A61F 2/385; A61F 2/3859;**
**A61F 2/3886; A61F 2/389;** A61F 2/30767;
A61F 2002/30011; A61F 2002/30332;
A61F 2002/30354; A61F 2002/30364;
A61F 2002/30375; A61F 2002/30405;
A61F 2002/30607; A61F 2002/30616;
A61F 2002/30878;                    (Cont.)

(84) Designated Contracting States:
**AL AT BE BG CH CY CZ DE DK EE ES FI FR GB**
**GR HR HU IE IS IT LI LT LU LV MC ME MK MT NL**
**NO PL PT RO RS SE SI SK SM TR**
Designated Extension States:
**BA**
Designated Validation States:
**KH MA MD TN**

(30) Priority: **22.12.2022 US 202263434573 P**

(71) Applicant: **Zimmer, Inc.**
**Warsaw, IN 46580 (US)**

(72) Inventors:
• **YOKO, Tim**
**Granger, IN 46530 (US)**
• **BARKER, Joshua**
**Warsaw, IN 46582 (US)**

(74) Representative: **Taor, Simon Edward William et al**
**Venner Shipley LLP**
**200 Aldersgate**
**London EC1A 4HD (GB)**

(54) **TIBIAL COMPONENT FOR A CONSTRAINED PROSTHETIC KNEE**

(57)     The techniques described herein relate to prosthesis assembly (122) including a baseplate (100) with a medial/lateral midline (A2) extending between a medial compartment (108) and a lateral compartment (110) and a keel (104) extending distally from the distal surface to define a longitudinal keel axis (LKA). The longitudinal keel axis is medially biased toward the medial compartment so as to be spaced a first distance (x1) medial of the medial/lateral midline. The prosthesis assembly includes a hinge post (126) having an longitudinal axis (LA), the hinge post received in a recess (148) in the baseplate including in the keel.

FIG. 1A

EP 4 389 080 A1

(52) Cooperative Patent Classification (CPC): (Cont.)
A61F 2002/3092; A61F 2002/3093;
A61F 2002/30985

**Description**

FIELD

The present subject matter relates to orthopedic prostheses and, more particularly, to tibial baseplates used in constrained knee arthroplasties.

BACKGROUND

Orthopedic procedures and prostheses are commonly utilized to repair and/or replace damaged bone and tissue in the human body. Generally, the knee is formed by the pair of condyles at the distal portion of the femur, the lower surfaces of which bear upon the correspondingly shaped proximal surface plateau of the tibia. The femur and tibia are connected by means of ligaments such as, the posterior cruciate ligament, the lateral collateral ligament, the medial collateral ligament, and the anterior cruciate ligament. These ligaments provide stability to the knee joint.

Prosthetic knee joints can be considered either constrained or unconstrained. For the purposes of this discussion, constrained prosthetic knee systems include femoral and tibial prostheses, which are mechanically linked or constrained to each other to limit relative movement between the femoral and tibial prostheses. Common mechanisms for such mechanical linkage is by a hinge, band or other linkage structure. An unconstrained prosthetic knee system includes femoral and tibial prostheses which are not mechanically linked. An unconstrained knee utilizes the patient's existing ligaments and other soft tissue to provide joint stability. With this in mind, constrained prosthetic knees have particular applicability to cases in which a patient has experienced ligament loss and/or the existing ligaments do not provide adequate support and stability to the knee.

Various constrained knee designs are known. One such design includes a hinge post. This hinge post configuration is positioned within a tibial baseplate (with an end protruding therefrom) and is connected to the femoral component. One hinge post configuration is the NexGen® Rotating Hinge Knee owned by the applicant, for example.

OVERVIEW

This disclosure pertains generally to improved constrained knee prostheses, particularly tibial baseplates thereof. It has been recognized for unconstrained prosthetic knee systems that having a medially biased keel can provide benefits including having the keel centered or nearly centered within the intramedullary canal. In this manner, the keel may avoid impinging onto hard, cortical bone around the intramedullary canal, thereby promoting firm and stable long-term fixation of the tibial baseplate to tibia. Furthermore, it has been recognized again for unconstrained prosthetic knee assemblies and systems that a degree of medialization of keel should increase as the tibial baseplates in the system become larger in size. However, medialization of the keel presents design challenges with addition of a hinge post configuration for a constrained knee system. In particular, for larger system sizes with a higher degree of medialization of the keel, if an axis of the hinge post is aligned with a medial/lateral midline of the tibial baseplate, an amount of material on a lateral side of the keel is reduced relative to a medial side. This reduced amount of material on the lateral side of the keel could lead to a possibility of a failure (breakout) of the hinge post from the keel for the larger system sizes with the higher degree of medialization. Thus, a reduction in the amount of material at the lateral side of the keel resulting from the hinge post being aligned with the medial/lateral midline of the tibial baseplate is not acceptable with larger sizes of tibial baseplates.

The present inventors have recognized, among other things, a medialized keel for the tibial baseplate can be achieved for a constrained knee system. Use of a medialized keel can avoid impinging onto hard, cortical bone around the intramedullary canal, thereby promoting firm and stable long-term fixation of tibial baseplate to tibia. The present inventors recognize several techniques that can allow for the medialized keel while also allowing for use of the hinge post configuration to mechanically link the femoral prosthesis and tibial baseplate together. As a first example, the present inventors have recognized that for all sizes of the tibial baseplate or at least for certain larger sizes of the tibial baseplate, a longitudinal axis defined by the hinge post can be medialized from the medial/lateral midline of the tibial baseplate.

According to other examples, which may or may not be combined with the example described above, the present inventors recognize that the femoral prosthesis (and/or other components of the prosthesis system) can be medialized with respect to the tibial baseplate. This can allow the hinge post to be medialized with respect to the medial/lateral midline of the tibial baseplate. Indeed, the present inventors have recognized that the hinge post can be medialized with respect to the medial/lateral midline of the tibial baseplate to such a degree that the hinge post received in the keel can be centralized with respect to the keel without adverse effects to knee joint kinematics. Alternatively, the present inventors recognize a design of the hinge post that creates a medial jog after the hinge post leaves the coupling point with the femoral component and enters the tibial baseplate. This jog can allow the portion of the hinge post received in the keel to be more centralized with respect to the keel even when the keel is medialized with respect to the medial/lateral midline of the tibial baseplate.

Alternatively, according to a second example, the present inventors recognize that a size of the keel can be increased to accommodate the hinge post (with the longitudinal axis of the hinge post positioned along the medial/lateral midline of the tibial baseplate). This increased size for the keel can be for the certain larger sizes of the tibial baseplate and can increase a thickness of the keel to provide for a relatively larger amount of material on the lateral side of the keel, thereby reducing the possibility of a failure in this region.

Additional features and benefits of the various examples provided herein will be discussed and/or will be apparent to one of ordinary skill in the art.

To further illustrate the apparatuses, systems and methods disclosed herein, the following non-limiting examples are provided, and which are referred to below as techniques. Parts or all of these examples/techniques can be combined in any manner.

In some aspects, the techniques described herein relate to a prosthesis assembly for a constrained knee including: a baseplate including: a medial compartment forming a first portion of a distal surface sized and shaped to substantially cover a first portion of proximal resected surface of a tibia and a first portion of a proximal surface opposite the first portion of the distal surface; a lateral compartment forming a second portion of the distal surface sized and shaped to substantially cover a second portion of proximal resected surface of a tibia and a second portion of the proximal surface opposite the second portion of the distal surface; a periphery extending around the medial compartment and the lateral compartment between the proximal surface and the distal surface; a medial/lateral midline extending between the medial compartment and the lateral compartment; and a keel extending distally from the distal surface to define a longitudinal keel axis, wherein longitudinal keel axis is medially biased toward the medial compartment so as to be spaced a first distance medial of the medial/lateral midline; and a hinge post having an longitudinal axis, the hinge post received in a recess in the baseplate including in the keel.

In some aspects, the techniques described herein relate to a prosthesis assembly, wherein the longitudinal axis of the hinge post is medially biased toward the medial compartment so as to be spaced a second distance medial of the medial/lateral midline.

In some aspects, the techniques described herein relate to a prosthesis assembly, wherein the first distance is substantially the same as the second distance.

In some aspects, the techniques described herein relate to a prosthesis assembly, wherein the longitudinal axis of the hinge post and the longitudinal keel axis are substantially coaxially aligned for at least three different standard stock sizes of the baseplate.

In some aspects, the techniques described herein relate to a prosthesis assembly, wherein the longitudinal axis of the hinge post is offset laterally from the longitudinal keel axis for at least three different standard stock sizes of the baseplate.

In some aspects, the techniques described herein relate to a prosthesis assembly, wherein a distance of the offset is between 0.25 mm and 2.5 mm, inclusive.

In some aspects, the techniques described herein relate to a prosthesis assembly, wherein the longitudinal axis of the hinge post is offset medially from the medial/lateral midline for all different standard stock sizes of the baseplate.

In some aspects, the techniques described herein relate to a prosthesis assembly, further including a femoral component connected to the hinge post, wherein a medial-lateral midline of the femoral component is offset medially with respect to the medial/lateral midline of the baseplate.

In some aspects, the techniques described herein relate to a prosthesis assembly, wherein a minimum wall thickness of the keel at a lateral side portion is at least 1.25 mm.

In some aspects, the techniques described herein relate to a prosthesis assembly, wherein a proximal end portion of the hinge post has a jog portion that extends medially from the proximal end portion toward a distal end portion of the hinge post.

In some aspects, the techniques described herein relate to a prosthesis assembly, wherein the hinge post is rotatable relative to the baseplate about the longitudinal axis.

In some aspects, the techniques described herein relate to a tibial prosthesis system including: a plurality of prostheses having a plurality of different standard stock sizes, each of the plurality of prostheses including: a baseplate including: a medial compartment forming a first portion of a distal surface sized and shaped to substantially cover a first portion of proximal resected surface of a tibia and a first portion of a proximal surface opposite the first portion of the distal surface; a lateral compartment forming a second portion of the distal surface sized and shaped to substantially cover a second portion of proximal resected surface of the tibia and a second portion of the proximal surface opposite the second portion of the distal surface; a periphery extending around the medial compartment and the lateral compartment between the proximal surface and the distal surface; a medial/lateral midline extending between the medial compartment and the lateral compartment; and a keel extending distally from the distal surface to define a longitudinal keel axis; one or more hinge posts having an longitudinal axis, the one or more hinge posts configured to be received in a recess in the baseplate including in the keel, wherein when the one or more hinge posts is received in the recess, the longitudinal axis of the hinge post and the longitudinal keel axis are substantially coaxially aligned for at least one of the plurality of the different standard stock sizes, and wherein the longitudinal axis of the hinge post is offset laterally from the longitudinal keel axis for two or more of the plurality of the different standard stock sizes.

In some aspects, the techniques described herein relate to a system, wherein longitudinal keel axis is medially biased toward the medial compartment so as to be spaced a first distance medial of the medial/lateral midline.

In some aspects, the techniques described herein relate to a system, wherein the first distance is between 0.25 mm and 2.5 mm, inclusive.

In some aspects, the techniques described herein relate to a system, wherein the longitudinal axis of the hinge post and the longitudinal keel axis are substantially coaxially aligned for at least three of the plurality of the different standard stock sizes.

In some aspects, the techniques described herein relate to a system, wherein the longitudinal axis of the hinge post is offset medially from the medial/lateral midline for all the plurality of different standard stock sizes.

In some aspects, the techniques described herein relate to a system, further including a femoral component connected to one of the one or more hinge posts, wherein a medial-lateral midline of the femoral component is offset medially with respect to the medial/lateral midline of the baseplate.

In some aspects, the techniques described herein relate to a system, wherein the longitudinal axis of the hinge post is offset laterally from the longitudinal keel axis for at least three of the plurality of the different standard stock sizes.

In some aspects, the techniques described herein relate to a system, wherein a proximal end portion of the one or more hinge posts has a jog portion that extends medially from the proximal end portion toward a distal end portion of the one or more hinge posts.

In some aspects, the techniques described herein relate to a system, wherein the one or more hinge posts is rotatable relative to the baseplate about the longitudinal axis of the hinge post when received in the recess.

In some aspects, the techniques described herein relate to a prosthesis assembly for a constrained knee including: a baseplate including: a medial compartment forming a first portion of a distal surface sized and shaped to substantially cover a first portion of proximal resected surface of a tibia and a first portion of a proximal surface opposite the first portion of the distal surface; a lateral compartment forming a second portion of the distal surface sized and shaped to substantially cover a second portion of proximal resected surface of a tibia and a second portion of the proximal surface opposite the second portion of the distal surface; a periphery extending around the medial compartment and the lateral compartment between the proximal surface and the distal surface; a medial/lateral midline extending between the medial compartment and the lateral compartment; and a keel extending distally from the distal surface to define a longitudinal keel axis, wherein longitudinal keel axis is medially biased toward the medial compartment so as to be spaced a first distance medial of the medial/lateral midline; a hinge post having an longitudinal axis, the hinge post received in a recess in the baseplate including in the keel, wherein the longitudinal axis of the hinge post is medially biased toward the medial compartment so as to be spaced a second distance medial of the medial/lateral midline; and a femoral component connected to the hinge post.

In some aspects, the techniques described herein relate to a prosthesis assembly, wherein at least one of: a medial-lateral midline of the femoral component is offset medially with respect to the medial/lateral midline of the baseplate; or a proximal end portion of the hinge post has a jog portion that extends medially from the proximal end portion toward a distal end portion of the hinge post.

In some aspects, the techniques described herein relate to a prosthesis assembly, wherein the first distance is substantially the same as the second distance.

In some aspects, the techniques described herein relate to a prosthesis assembly, wherein the longitudinal axis of the hinge post and the longitudinal keel axis are substantially coaxially aligned for at least three different standard stock sizes of the baseplate.

In some aspects, the techniques described herein relate to a prosthesis assembly, wherein the longitudinal axis of the hinge post is offset laterally from the longitudinal keel axis for at least three different standard stock sizes of the baseplate.

In some aspects, the techniques described herein relate to a prosthesis assembly, wherein a distance of the offset is between 0.25 mm and 2.5 mm, inclusive.

In some aspects, the techniques described herein relate to a prosthesis assembly, wherein the longitudinal axis of the hinge post is offset medially from the medial/lateral midline for all different standard stock sizes of the baseplate.

In some aspects, the techniques described herein relate to a prosthesis assembly, wherein a minimum wall thickness of the keel at a lateral side portion is at least 1.25 mm.

BRIEF DESCRIPTION OF THE DRAWINGS

In the drawings, which are not necessarily drawn to scale, like numerals can describe similar components in different views. Like numerals having different letter suffixes can represent different instances of similar components. The drawings illustrate generally, by way of example, but not by way of limitation, various examples discussed in the present document.

FIG. 1 shows an example of distal side of a first tibial baseplate for a constrained knee prosthesis system or assembly with a keel that is medialized such that a longitudinal keel axis is offset medially relative to a medial/lateral centerline of the baseplate according to an example of the present application.

FIG. 1A shows a cross-sectional view along a coronal plane showing the first tibial baseplate of FIG. 1A and additionally a hinge post and tibial bearing with a longitudinal axis of the hinge post substantially coaxially aligned with the medial/lateral centerline of the baseplate according to an example of the present application.

FIG. 2 is a perspective view of a constrained knee prosthesis assembly according to an example of the present application.

FIG. 3 is an exploded view of the constrained knee prosthesis assembly of FIG. 2.

FIG. 4 shows a plurality of tibial baseplates having a plurality of different standard stock sizes that are used as part of a prosthesis system according to an example of the present application.

FIG. 4A is a chart showing an offset distance in a medial direction for the longitudinal keel axis and an offset distance in a medial direction for the longitudinal axis of the hinge post relative to the medial/lateral centerline of the different stock sizes of baseplate shown in FIG. 4.

FIG. 5 shows a proximal side of a large stature tibial baseplate of FIG. 4 having a recess therein, FIG. 5 further illustrates the medial/lateral centerline of the baseplate relative to the longitudinal keel axis and the longitudinal axis of the hinge post that results from the hinge post being medialized relative to the medial/lateral centerline of the baseplate according to an example of the present application.

FIG. 5A is an enlarged view of a portion of the tibial baseplate of FIG. 5.

FIG. 6 shows two cross-sectional views in a coronal plane of the keel and hinge post, the image on the viewer's right has additional material on a lateral side of the keel that results from the hinge post being medialized or thickened according to techniques of the present application.

FIG. 7A is a cross-sectional view of the constrained knee prosthesis assembly in a coronal plane with the hinge post centralized such that the longitudinal axis of the hinge post is coaxially aligned with the medial/lateral centerline of the baseplate according to an example of the present application.

FIG. 7B is a cross-sectional view of the constrained knee prosthesis assembly of FIG. 7A but with the femoral component and/or other components medialized such that a medial/lateral centerline of the femoral component is offset from the medial/lateral centerline of the baseplate.

FIG. 8 is a cross-sectional view of another constrained knee prosthesis assembly in a coronal plane with a hinge post having a jog portion that extends medially from a proximal end portion toward a distal end portion of the hinge post.

FIG. 9 is a cross-sectional view of the constrained knee prosthesis assembly with the femoral component, tibial bearing component and/or other components are medialized relative to the tibial baseplate such that the longitudinal axis of the hinge post is coaxially aligned with the longitudinal keel axis of the keel according to an example of the present application.

DETAILED DESCRIPTION

The present application relates to constrained tibial prosthesis assemblies and systems including tibial baseplates and a hinge post among other components. This application focuses on positioning of the hinge post relative to the keel of the tibial baseplate and other solutions for increasing an amount of material on a lateral side of the keel when the hinge post and keel are medialized relative to the remainder of the baseplate as further discussed herein. As discussed previously, proper positioning of the keel with respect to the intermedullary canal of the tibia can improve fixation and the durability of the tibial baseplate among other benefits.

As used herein, the terms "proximal" and "distal" should be given their generally understood anatomical interpretation. The term "proximal" refers to a direction generally toward the torso of a patient, and "distal" refers to the opposite direction of proximal, i.e., away from the torso of a patient. It should be understood that the use of the terms "proximal" and "distal" should be interpreted as though the patient were standing with the knee joint in extension despite the apparatuses described herein generally being used with the knee joint in flexion. The intent is to differentiate the terms "proximal" and "distal" from the terms "anterior" and "posterior". As used herein, the terms "anterior" and "posterior" should be given their generally understood anatomical interpretation. Thus, "posterior" refers to a rear of the patient, e.g., a back of the knee. Similarly, "anterior" refers to a front of the patient, e.g., a front of the knee. Thus, "posterior" refers to the opposite direction of "anterior". Similarly, the term "lateral" refers to the opposite direction of "medial". The term "medial/lateral" means medial to lateral or lateral to medial. The term "proximal/distal" means proximal to distal or distal to proximal. The term "anterior/posterior" means anterior to posterior or posterior to anterior.

As used herein, the "periphery" of a tibial baseplate refers to any periphery as viewed in a top plan view, e.g., in a generally transverse anatomical plane. Alternatively, the periphery of a tibial baseplate may be any periphery as viewed in bottom plan view, e.g., in a generally transverse plane and looking at the distal surface adapted to contact a resected proximal surface of a tibial bone. In the context of a prosthesis, such as tibial baseplate described below, the medial/lateral centerline (sometimes referred to a "home axis" or "anteroposterior axis") refers to an axis that divides a lateral compartment of the baseplate from a medial compartment. The medial/lateral centerline can extend anterior/posterior (e.g., from an anterior periphery of the baseplate to a posterior periphery of the baseplate) as the medial/lateral centerline extends generally anteriorly and posteriorly when baseplate is implanted upon tibia. The medial/lateral centerline can be positioned equidistant from a medial most point and lateral most point along the periphery. A typical practice upon implantation of the baseplate is to have the baseplate oriented relative to the tibia such that the medial/lateral centerline is substantially aligned with a home axis of tibia. Such orientation allows for a proper rotational and spatial orientation. However, it is contemplated that medial/lateral centerline may be oriented other manners relative to the home axis of tibia. As described below, distal features such as the keel can be oriented relative to the medial/lateral centerline, etc.

The home axis of the tibia can extend from a posterior point to an anterior point. The posterior point is generally disposed in the area where the patient's posterior cruciate ligament (PCL) attaches or would attach to tibia. More specifically, the posterior point is generally disposed at the geometric center of the attachment between the patient's PCL and tibia. The patient's PCL typically attaches to tibia T in two ligament "bundles," the first bundle having a more anterolateral attachment location and the second bundle having a more posteromedial attachment location. Although the posterior point PP is described at the geometric center of the first bundle, it is contemplated that the posterior point may be located at the geometric center of the second bundle or at the geometric center of the first and second bundles, together. The anterior point can be disposed on the patient's anterior tibial tubercle. The anterior point can be medially spaced from the tubercle midpoint by an amount equal to $\frac{1}{6}$ of the overall medial/lateral tubercle width. Stated another way, the anterior point can be laterally spaced from the tubercle medial end by an amount equal to ⅓ of the overall medial/lateral tubercle width, such that the anterior point lies on the "medial third" of the anterior tibial tubercle.

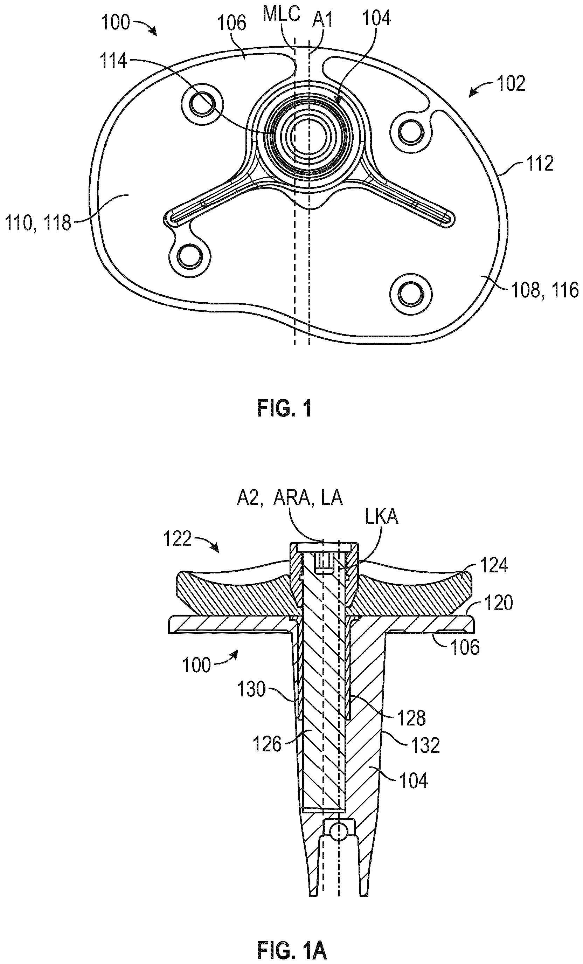

FIG. 1 shows a plan view of a distal side 102 of a tibial baseplate 100. The tibial baseplate 100 includes a keel 104, a distal surface 106, a medial compartment 108, a lateral compartment 110 and a periphery 112.

The tibial baseplate 100 can be used as part of a constrained knee prosthesis system or assembly as further discussed herein. The keel 104 extends distally from the distal surface 106 of the tibial baseplate 100. The keel 104 can be integral with (monolithic) or can be attached to (e.g. via thread or another mechanical connection mechanism) a remainder of the tibial baseplate 100. It is contemplated that a core 114 of the keel 104 may have a substantially conical, or have a tapered outer profile. The taper angle may be formed, for example, by tapering the core 114 of the keel 104 from a larger outer diameter at a proximal terminus of keel 104 (i.e., at the junction between keel 104 and distal surface 106 of tibial baseplate 100) to a relatively smaller distal diameter of at the distal terminus of keel 104 at a tip thereof. Exemplary constructs for the keel are discussed in United States Patent Nos. 8,758,444B2 and 9,381,090B2, the entire specification of each of which is incorporated by reference in its entirety.

The keel 104 can be configured to extend distally and can be shaped to fit in an intermedullary canal of a tibia (not show) to provide fixation for the tibial baseplate 100. The keel 104 can include features that extend from the core 114 such as fins and can have a distal aperture to receive a stem extension as known in the art. As shown in FIG. 1, the keel 104 can be medialized in orientation. In particular, the keel 104 can be positioned such that at least a majority of the keel 104 is positioned on and extends from the medial compartment 108. Thus, relatively less of the keel 104 is positioned on and extends from the lateral compartment 110 as compared with the medial compartment 108.

FIG. 1A shows a longitudinal extent of the keel 104 extending distally from the distal surface 106 to a tip. The keel 104 defines a longitudinal keel axis LKA. This longitudinal keel axis LKA can comprise a centerline of the keel 104. The keel 104 can be symmetric in shape about the longitudinal keel axis LKA. However, according to other examples the keel 104 can be asymmetric in shape but can still have the longitudinal keel axis LKA. FIG. 1 shows an axis A1 that is orthogonal to the longitudinal keel axis LKA of FIG. 1A. Thus, axis A1 approximates the location of the longitudinal keel axis LKA for viewer reference in FIG. 1.

Referring again to FIG. 1, the medial compartment 108 can oppose the lateral compartment 110. The transition between the medial compartment 108 and the lateral compartment 110 occurs at a medial/lateral centerline MLC, which is indicated as an axis extending generally anterior/posterior across the tibial baseplate 100 from a first side of the periphery 112 to a second side of the periphery 112. The medial/lateral centerline MLC divides the tibial baseplate 100 into the medial compartment 108 and the lateral compartment 110. Thus, the medial/lateral centerline MLC extends between the medial compartment 108 and the lateral compartment 110. As shown in FIG. 1, the axis A1 (approximating the position of the longitudinal keel axis LKA) extends parallel with but spaced a distance from the medial/lateral centerline MLC. This is due to the medialization of the keel 104. FIG. 1A shows an axis A2 that is orthogonal to the medial/lateral centerline MLC. The axis A2 (approximating the position of the medial/lateral centerline MLC) extends parallel with but is spaced laterally from the longitudinal keel axis LKA. The positioning of the axis A2 relative to the longitudinal keel axis LKA is due to the medialization of the keel 104.

Due to the medialization of the keel 104, a majority of the keel 104 is positioned medial of the medial/lateral centerline MLC and the axis A1 as shown in FIG. 1. This positioning of the keel 104 results in the longitudinal keel axis LKA being medial of and spaced a distance from the axis A2 as shown in FIG. 1A.

The distal surface 106 can include features such as threaded apertures for the connection of pegs, augments or other components as known in the art. The tibial baseplate 100, and hence the distal surface 106, can have a particular asymmetry, with respect to the medial/lateral centerline MLC. This shape is designed to maximize tibial coverage for a large proportion of knee-replacement candidates. The asymmetric shape results in the medial compartment 108 being relatively larger than the lateral compartment 110. Maximized coverage of cortical bone facilitates superior support of tibial baseplate 100. A firm, enduring fixation of tibial baseplate 100 to tibia is facilitated by large-area contact between the cortical and cancellous bone of tibia.

Distal surface 106 (and other features of the tibial baseplate such as the keel 104) can be made of a porous or highly porous material that facilitates an amount of bone ingrowth. A highly porous biomaterial is useful as a bone substitute and as cell and tissue receptive material. A highly porous biomaterial may have a porosity as low as 30%, 55%, or as high as 70%, 80%, 85%, or 90%. The highly porous material can have an average pore size of between 100 microns and 1000 microns, for example. However, use of the highly porous biomaterial is not contemplated in all examples. For example, material such as bone cement can be utilized as an alternative to the highly porous biomaterial.

An example of such porous or highly porous material is OsseoTi® generally available from Zimmer Biomet, Inc., of Warsaw, Ind. The material can include titanium or titanium alloy and can additionally include other materials. Such material (including a base of relatively less porous or non-porous biocompatible material) can be manufactured using additive manufacturing processes such as laser sintering or the like. OsseoTi® is highly biocompatible, has high corrosion resistance and includes a highly interconnected porous architecture that mimics the porous structure of human cancellous bone, which can enhance bone integration and in-growth. The porous or highly porous material can be manufactured to be layered over or otherwise structured with/on a relatively less porous or non-porous biocompatible material such as titanium, titanium alloy, stainless steel or other material as known in the art.

Another example of such a porous or highly porous material is produced using Trabecular Metal™ Technology generally available from Zimmer Biomet, Inc., of Warsaw, Ind. Such a material may be formed from a reticulated vitreous carbon foam substrate which is infiltrated and coated with a biocompatible metal, such as tantalum, by a chemical vapor deposition ("CVD") process in the manner disclosed in detail in U.S. Pat. No. 5,282,861 to Kaplan, the entire disclosure of which is hereby expressly incorporated herein by reference. In addition to tantalum, other metals such as niobium, or alloys of tantalum and niobium with one another or with other metals may also be used. The porous tantalum structure may be made in a variety of densities in order to selectively tailor the structure for particular applications. In particular, as discussed in the above-incorporated U.S. Pat. No. 5,282,861, the porous tantalum may be fabricated to virtually any desired porosity and pore size, and can thus be matched with the surrounding natural bone in order to provide an improved matrix for bone ingrowth and mineralization.

Generally, the porous material structures contemplated can include a large plurality of ligaments defining open spaces there between, with each ligament generally including a core covered by a thin film of metal. The open spaces between the ligaments form a matrix of continuous channels having no dead ends, such that growth of cancellous bone through the porous tantalum structure is uninhibited. The porous or highly porous material may include up to 70%, 85%, or more void space therein. Thus, porous or highly porous material is a lightweight, strong porous structure which is substantially uniform and consistent in composition, and closely resembles the structure of natural cancellous bone, thereby providing a matrix into which cancellous bone may grow to provide fixation of the tibial baseplate 100 to the patient's bone.

The medial compartment 108 forms a first portion 116 of the distal surface 106. The first portion 116 can be sized and shaped to substantially cover a first portion of proximal resected surface of the tibia. Similarly, the lateral compartment 110 forms a second portion 118 of the distal surface 106. The second portion 118 can be sized and shaped to substantially cover a second portion of proximal resected surface of the tibia.

The periphery 112 extends around the medial compartment 108 and the lateral compartment 110 between the proximal surface 120 (shown in FIG. 1A but not shown in FIG. 1) and the distal surface 106.

FIG. 1A shows additional features of a prosthesis assembly 122 for a constrained knee in addition to the tibial baseplate 100. These include a tibial bearing component 124 (sometime called a meniscal component, poly, articular component or bearing), a hinge post 126 and a bushing 128. The tibial bearing component 124 can be positioned atop the proximal surface 120 and can be formed of polymer material such as Ultra-High-Molecular-Weight-Polyethylene ("UHMWPE"), etc. The tibial bearing component 124 can be configured to articulate with a femoral component (not shown). As shown in FIG. 1A, the offset of the keel 104 medially (sometimes called a medial bias or medially biased herein) relative to the hinge post 126 results in an amount of material on a lateral side 130 of the keel 104 being reduced relative to a medial side 132. This reduced material on the lateral side 130 occurs because a longitudinal axis LA of the hinge post 126 is aligned or substantially aligned with the medial/lateral midline (approximated by axis A2 in FIG. 1A) of the tibial baseplate 100. The longitudinal axis LA of the hinge post 126 that approximates a centerline of the hinge post 126, for example. According to some examples, the longitudinal axis LA can be an axis of rotation and/or articulation ARA (simply referred to as the axis of rotation/articulation ARA herein) for the hinge post 126 relative to the tibial baseplate 100. However, according to further examples the hinge post 126 can be constrained so as not to rotate relative to the tibial baseplate 100 but can still provide an axis for articulation of the tibial poly on the femoral component. As discussed, a relatively thin region of material at the lateral side 130 of the keel 104 can be undesirable, particularly if the bushing 128 is utilized. The present application presents several solutions to increase the amount of material on the lateral side 130 of the keel 104.

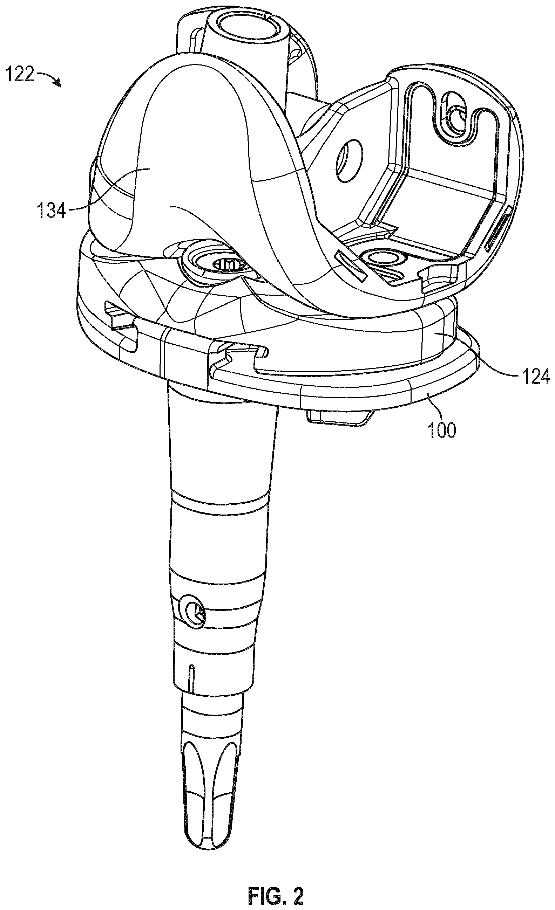

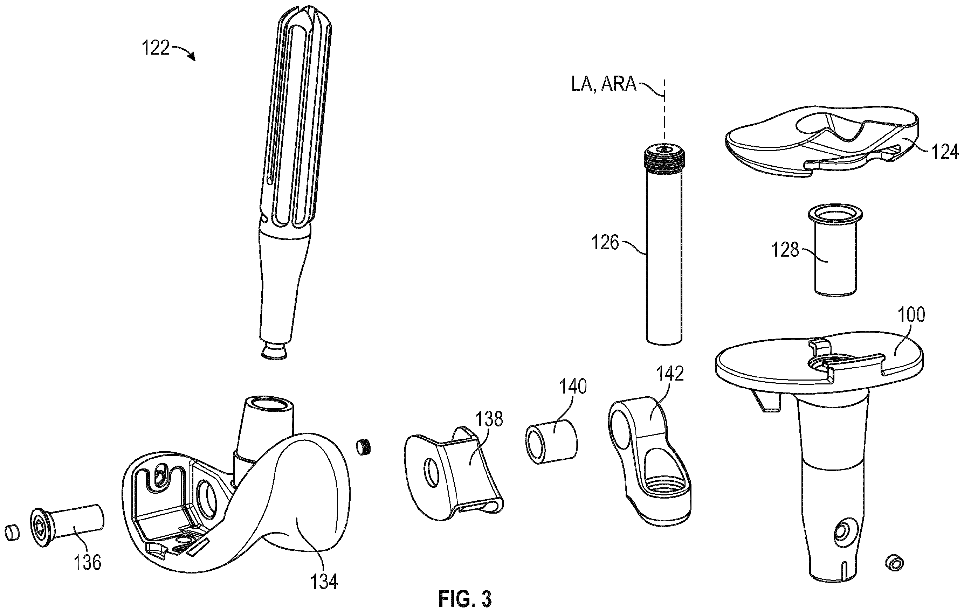

FIG. 2 shows the prosthesis assembly 122 for a constrained knee arthroplasty including the tibial baseplate 100, the tibial bearing component 124 and a femoral component 134. FIG. 3 shows an exploded view of the prosthesis assembly 122 illustrating further components including a hinge axle 136, poly box 138, axle bushing 140, shackle 142 and the hinge post 126 and the bushing 128 (discussed and shown previously in reference to FIG. 1A).

The prosthesis assembly 122 has the femoral component 134 and the tibial baseplate 100 mechanically linked to one another. This is accomplished by the hinge post 126 and other components of FIG. 3. In particular, the hinge post 126 is connected to femoral component 134 via the shackle 142, the axle bushing 140 and the hinge axle 136. A distal portion of the shackle 142 is received in a recess in the tibial bearing component 124 and this distal portion is threaded or otherwise connected to the hinge post 126. The hinge post 126 extends distally and can be received in a recess of the tibial baseplate 100 and a recess of the tibial bearing component 124. The recess of the tibial baseplate 100 that receives the hinge post 126 is at least partially formed by the keel 104. The hinge post 126 can be moveable (e.g., rotatable and/or capable of distraction) relative to one or more of the tibial bearing component 124 and/or the tibial baseplate 100. The hinge post 126 can be rotatably connected to femoral component 134 via the hinge axle 136. Thus, the hinge post 126 can define the axis of rotation/articulation ARA for the knee joint as the femoral component 134 and the tibial baseplate 100 are mechanically linked.

When assembled, the shackle 142 can be placed between opposing walls of poly box 138. When assembled on the hinge axle 136, the axle bushing 140 additionally resides within an aperture on a proximal portion of the shackle 142. The shackle 142 and hinge post 126 can be formed from suitable materials such as a titanium alloy, a cobalt-chromium alloy, etc. while the axle bushing 140 and the poly box 138 can be formed from a different materials such as plastic, e.g., UHMWPE. The axle bushing 140 acts as a bearing between the shackle 142 and the hinge axle 136. The poly box 138 acts as a bearing between the femoral component 134 and the shackle 142.

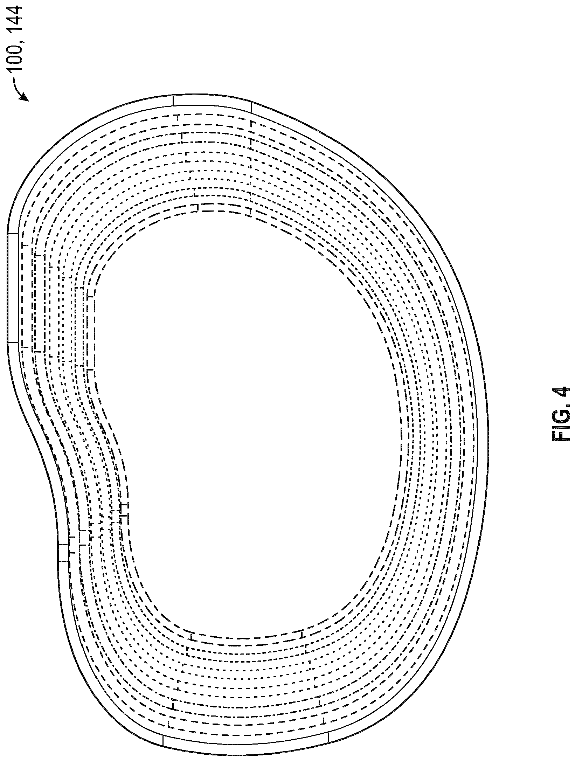

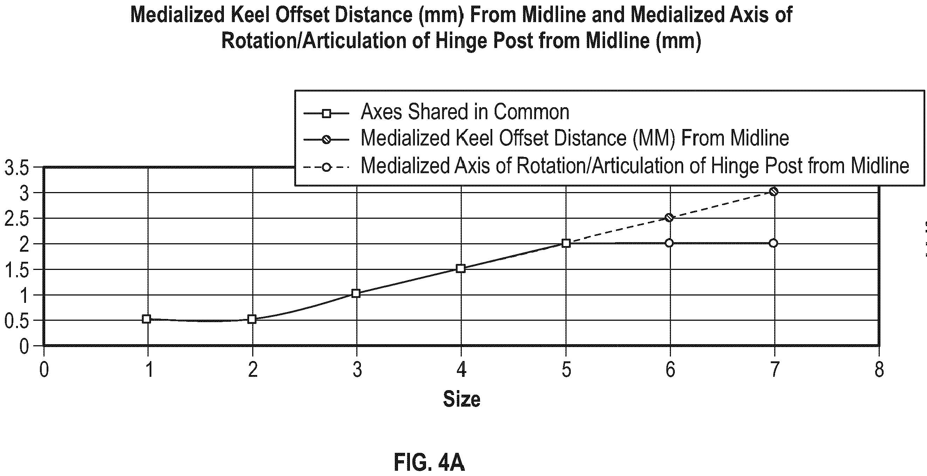

FIG. 4 shows the tibial baseplate 100 can be available in a prosthesis system 144 that includes plurality of different standard stock sizes (size 1 to size 7 as an example) for the tibial baseplate 100. These different standard sizes are selected to provide coverage to various sizes of tibia. As an example, the tibial baseplate 100 can include seven sizes from a size 1 (smallest stature) to a size 7 (largest stature). The various sizes can be available as the prosthesis system 144 to a surgeon to make a proper size election. The standard sizes have different geometries including along the proximal side (e.g., periphery 112, proximal surface, etc.) and distal side (e.g., distal surface 106, keel 104, etc.) as previously shown in FIGS. 1-3 and further described herein. It should be noted that as the stock size of the tibial baseplate 100 increases, the distance of the offset created between the longitudinal keel axis LKA and the medial/lateral centerline MLC can increase in a linear or non-linear manner. Thus, as an example for a size 1 tibial baseplate 100, the distance of the medial offset between the longitudinal keel axis LKA and the medial/lateral centerline MLC can be 0.5 mm (see FIG. 4A and TABLE 1). The distance of the medial offset between the longitudinal keel axis LKA and the medial/lateral centerline MLC can be 3.0 mm for a size 7 tibial baseplate.

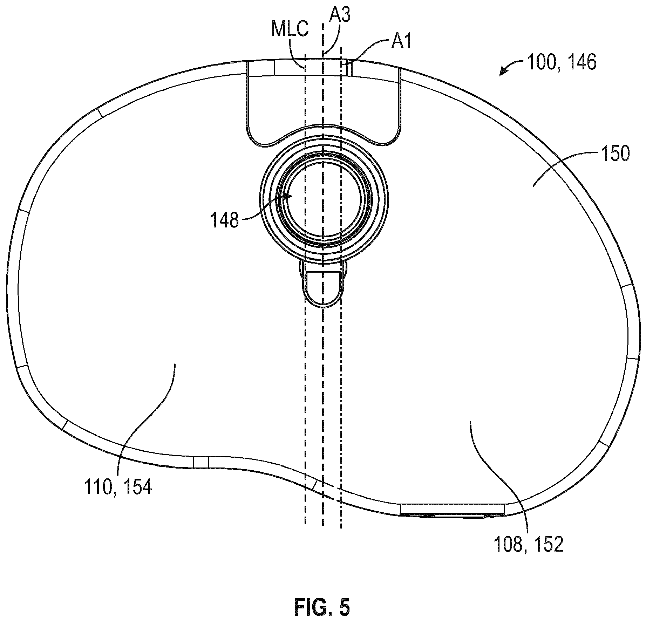

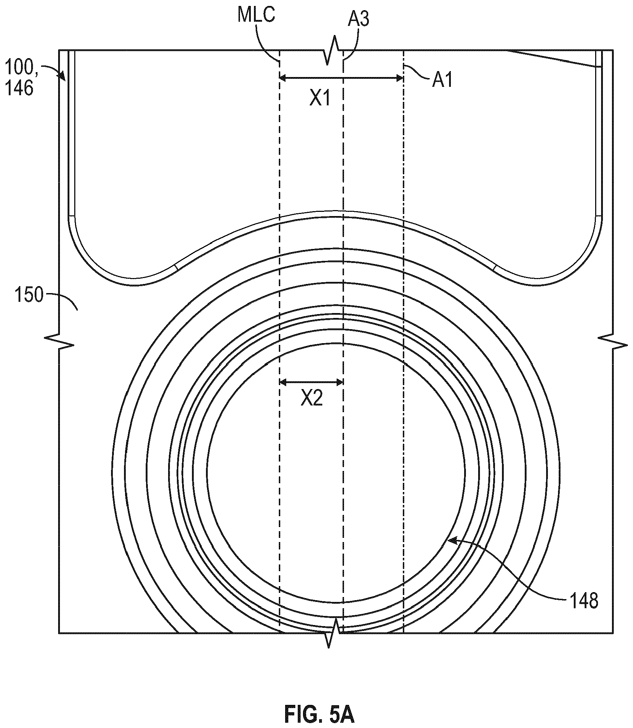

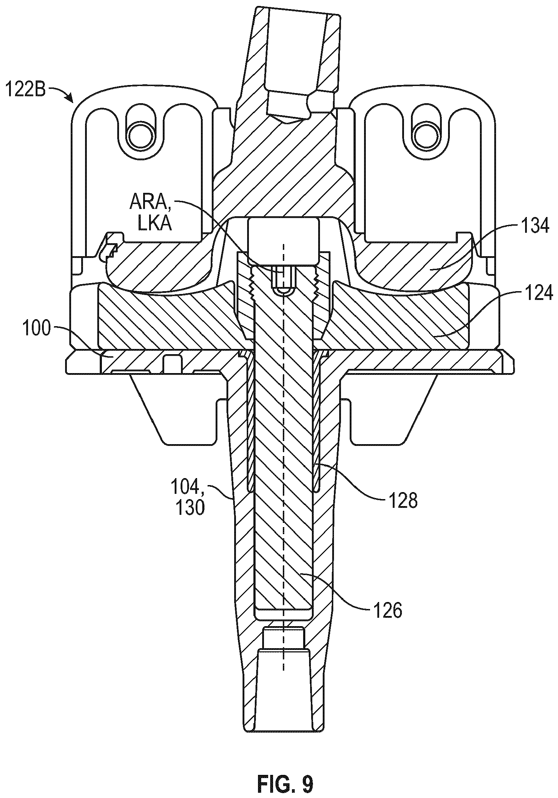

Similarly, there can be a distance of medial offset between the longitudinal axis LA (including the axis of rotation/articulation ARA in the examples of FIGS. 2-6) of the hinge post and the medial/lateral centerline MLC. This distance of medial offset between the longitudinal axis LA of the hinge post and the medial/lateral centerline MLC can be substantially the same as the distance of the offset created between the longitudinal keel axis LKA and the medial/lateral centerline MLC for three or more (or indeed for all different standard stock sizes in the example shown in FIG. 9) of the different stock sizes. However, for at least a portion of the different standard stock sizes, the distance of medial offset between the longitudinal axis LA of the hinge post and the medial/lateral centerline MLC can differ from the distance of medial offset between the longitudinal keel axis LKA and the medial/lateral centerline MLC. Thus, for example, for a size 7, the medial offset between the longitudinal keel axis LKA and the medial/lateral centerline MLC can be 3.0 mm but the medial offset between the longitudinal axis LA of the hinge post and the medial/lateral centerline MLC can be 2.0 mm. This provides an additional 1.0 mm of material on a lateral side of the keel for the size 7 tibial baseplate 100. Put another way, this difference in the two offsets provides for additional material on the lateral side of the keel in the examples of FIGS. 5-6 but this additional 1.0 mm of material is not present on the lateral side 130 in the example of FIG. 1A.

TABLE 1

| Size | Medialized Keel Offset Distance From Midline | Offset Distance of Hinge Post from Midline |
|---|---|---|
| 1 | 0.5 mm | 0.5 mm |
| 2 | 0.5 mm | 0.5 mm |
| 3 | 1.0 mm | 1.0 mm |
| 4 | 1.5 mm | 1.5 mm |
| 5 | 2.0 mm | 2.0 mm |
| 6 | 2.5 mm | 2.0 mm |
| 7 | 3.0 mm | 2.0 mm |

FIG. 4A shows a chart of the different medial offsets as described in TABLE 1.

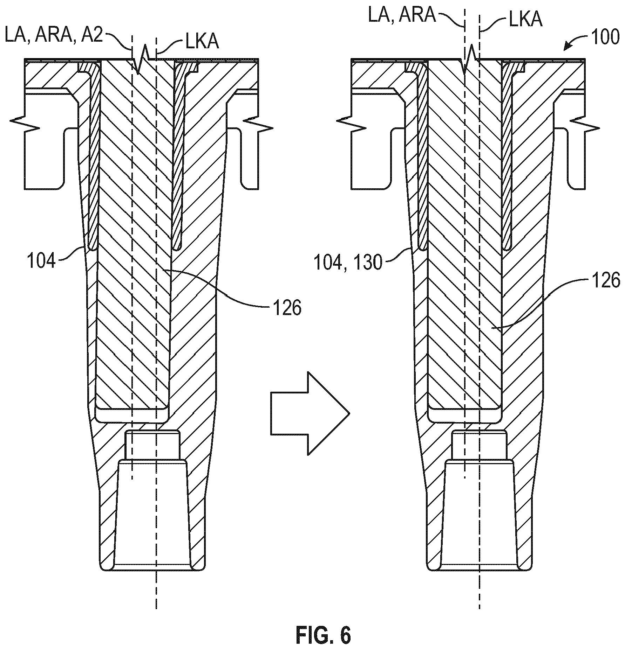

FIG. 5 shows a proximal side 146 of a large stature standard size of the tibial baseplate 100 with the medial/lateral centerline MLC, the axis A1 that is orthogonal to the longitudinal keel axis LKA (FIG. 6) and an axis A3 that is orthogonal to the longitudinal axis LA of the hinge post 126 (FIG. 6). FIG. 5A is an enlarged view of a portion of the proximal side 146 of the large stature standard size of the tibial baseplate 100 of FIG. 5.

As shown in FIGS. 5 and 5A, a recess 148 in a proximal surface 150 of the tibial baseplate 100 can be configured to receive the hinge post 126 (FIG. 6). This recess 148 can extend into the keel 104 as shown in FIG. 6. FIG. 5 shows the medial compartment 108 from the proximal side 146. The medial compartment 108 can include a first portion 152 of the proximal surface 150. This first portion 152 can be opposite the first portion 116 of the distal surface 106 (FIG. 1). Similarly, the lateral compartment 110 can include a second portion 154 of the proximal surface 150. The second portion 154 can be opposite the second portion 118 of the distal surface 106 (FIG. 1).

As shown in FIG. 5A, the axis A1 approximating the longitudinal keel axis LKA is offset a distance X1 medial from the medial/lateral centerline MLC. The axis A3 approximating the longitudinal axis LA of the hinge post 126 (FIG. 6) can be offset a distance X2 medial from the medial/lateral centerline MLC. For the larger stature tibial baseplate 100 (e.g., size 7 or 8) shown in FIG. 5A, the distance X1 can be larger than the distance X3. This difference in the medialization can be on the order of about 0.1 mm to about 2.5 mm, for example.

FIG. 6 illustrates on the viewers right the tibial baseplate 100 of FIGS. 5 and 5A. FIG. 6 shows the difference in the medialization between the longitudinal axis LA of the hinge post 126 and the longitudinal keel axis LKA (with the longitudinal axis LA of the hinge post 126 lateral of the longitudinal keel axis LKA) can provide for additional material at the lateral side 130 of the keel 104 (as seen with the keel 104 and hinge post 126 arrangement to the viewer's right) as compared with an arrangement where the longitudinal axis LA of the hinge post 126 and the axis A2 (approximating the medial/lateral centerline MLC of FIGS. 5 and 5C) are substantially coaxially aligned (as seen with the keel 104 and hinge post 126 arrangement to the viewer's left).

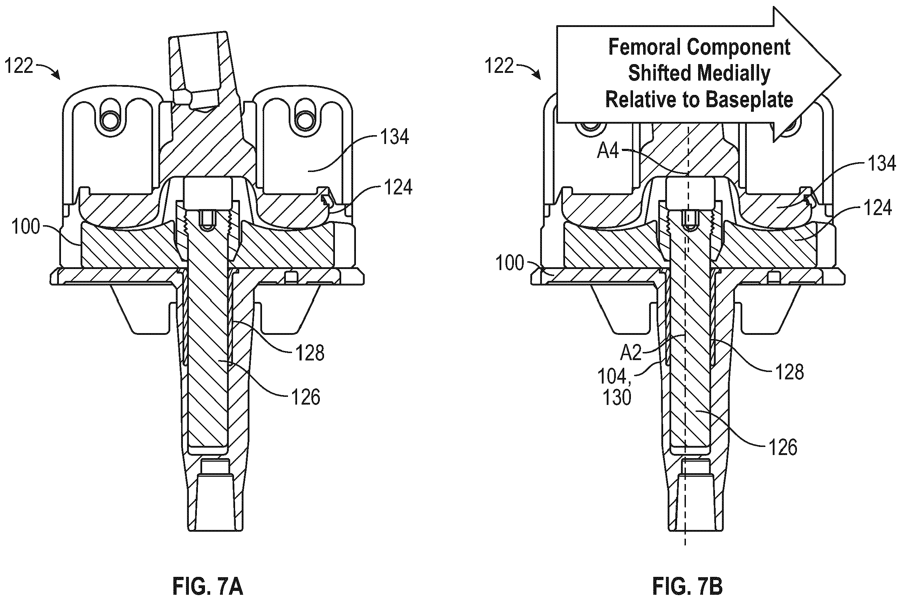

FIGS. 7A and 7B show the prosthesis assembly 122 for the constrained knee described previously with regard to FIGS. 2-3. Thus, the tibial baseplate 100, tibial bearing component 124, the hinge post 126, the bushing 128 and the femoral component 134 are illustrated.

FIG. 7B shows an example where components of the prosthesis assembly 122 including the tibial bearing component 124, the hinge post 126, the bushing 128 and/or the femoral component 134 are shifted medially in position relative to the tibial baseplate 100. This medial shift can be on the order of about 0.25 mm to about 3.5 mm, inclusive. The medial shift of the tibial bearing component 124, the hinge post 126, the bushing 128 and/or the femoral component 134 relative to the tibial baseplate 100 can allow for additional material at the lateral side 130 of the keel 104 as seen in FIG. 7B as compared with FIG. 7A. Thus, the minimum thickness of the keel 104 at the lateral side 130 can be greater than 1.25 mm for example in FIG. 7B. A result of the medializing shown in FIG. 7B, can include a medial/lateral centerline (approximated by axis A4 in FIG. 7B) of the femoral component 134 is offset from the medial/lateral centerline of the tibial baseplate 100 (as approximated by the axis A2).

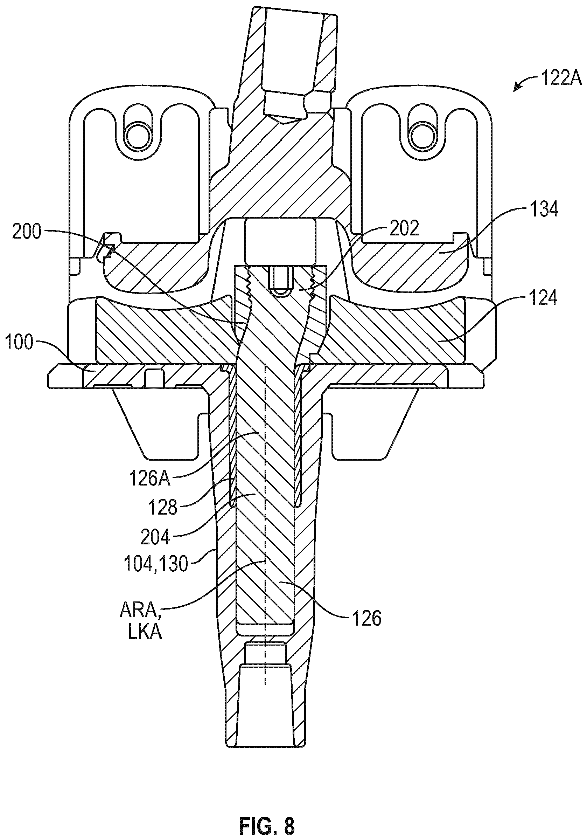

FIG. 8 shows a prosthesis assembly 122A similar to the prosthesis assembly 122 described previously. The prosthesis assembly 122A differs in that a hinge post 126A having a jog portion 200 is utilized. More particularly, a proximal end portion 202 of the hinge post 126A has the jog portion 200. The proximal end portion can be received in and coupled to the femoral component 134, for example. The jog portion 200 extends medially from the proximal end portion 202 toward a distal end portion 204 of the hinge post 126A. The distal end portion 204 can be received in the recess in the keel 104. This configuration for the hinge post 126A allows the longitudinal axis LA of the hinge post 126A and the longitudinal keel axis LKA to be substantially coaxially aligned within the keel 104. Thus, the hinge post 126A within the keel 104 (the distal end portion 204) can have a centralized in positioning with the lateral side 130 of the keel 104 having a substantially same thickness as the medial side 132 of the keel 104. The tibial bearing component 124 and/or the femoral component 134 need not be medialized relative to the tibial baseplate 100 with the prosthesis assembly 122A of FIG. 8.

FIG. 9 shows a prosthesis assembly 122B similar to the prosthesis assembly 122 described previously in FIG. 8 where components such as the tibial bearing component 124, the hinge post 126, the bushing 128 and/or the femoral component 134 are shifted medially relative to the tibial baseplate 100. The prosthesis assembly 122B differs from FIG. 8 in that the amount of the medial shift allows the longitudinal axis LA of the hinge post 126 and the longitudinal keel axis LKA to be substantially coaxially aligned. The hinge post 126 within the keel 104 can be centralized in positioning with the lateral side 130 of the keel 104 having a substantially same thickness as the medial side 132 of the keel 104. The hinge post 126 need not utilize a jog portion as was the case with the example of FIG. 8.

Additional Notes

The above detailed description includes references to the accompanying drawings, which form a part of the detailed description. The drawings show, by way of illustration, specific embodiments in which the invention can be practiced. These embodiments are also referred to herein as "examples." Such examples can include elements in addition to those shown or described. However, the present inventors also contemplate examples in which only those elements shown or described are provided. Moreover, the present inventors also contemplate examples using any combination or permutation of those elements shown or described (or one or more aspects thereof), either with respect to a particular example (or one or more aspects thereof), or with respect to other examples (or one or more aspects thereof) shown or described herein.

In this document, the terms "generally" "substantially" "about" mean within 15 percent of the value provided ($\pm$). The terms "a" or "an" are used, as is common in patent documents, to include one or more than one, independent of any other instances or usages of "at least one" or "one or more." In this document, the term "or" is used to refer to a nonexclusive or, such that "A or B" includes "A but not B," "B but not A," and "A and B," unless otherwise indicated. In this document, the terms "including" and "in which" are used as the plain-English equivalents of the respective terms "comprising" and "wherein." Also, in the following claims, the terms "including" and "comprising" are open-ended, that is, a system, device, article, composition, formulation, or process that includes elements in addition to those listed after such a term in a claim are still deemed to fall within the scope of that claim. Moreover, in the following claims, the terms "first," "second," and "third," etc. are used merely as labels, and are not intended to impose numerical requirements on their objects.

The above description is intended to be illustrative, and not restrictive. For example, the above-described examples (or one or more aspects thereof) can be used in combination with each other. Other examples can be used, such as by one of ordinary skill in the art upon reviewing the above description. The Abstract is provided to comply with 37 C.F.R. §1.72(b), to allow the reader to quickly ascertain the nature of the technical disclosure. It is submitted with the understanding that it will not be used to interpret or limit the scope or meaning of the claims. Also, in the above detailed description, various features can be grouped together to streamline the disclosure. This should not be interpreted as intending that an unclaimed disclosed feature is essential to any claim. Rather, inventive subject matter can lie in less than all features of a particular disclosed example. Thus, the following claims are hereby incorporated into the detailed description as examples or embodiments, with each claim standing on its own as a separate example, and it is contemplated that such examples can be combined with each other in various combinations or permutations. The scope of the invention should be determined with reference to the appended claims, along with the full scope of equivalents to which such claims are entitled.

**Claims**

1.    A prosthesis assembly for a constrained knee comprising:

   a baseplate comprising:

      a medial compartment forming a first portion of a distal surface sized and shaped to substantially cover a first portion of proximal resected surface of a tibia and a first portion of a proximal surface opposite the first portion of the distal surface;
      a lateral compartment forming a second portion of the distal surface sized and shaped to substantially cover a second portion of proximal resected surface of a tibia and a second portion of the proximal surface opposite the second portion of the distal surface;
      a periphery extending around the medial compartment and the lateral compartment between the proximal surface and the distal surface;

a medial/lateral midline extending between the medial compartment and the lateral compartment; and

a keel extending distally from the distal surface to define a longitudinal keel axis, wherein longitudinal keel axis is medially biased toward the medial compartment so as to be spaced a first distance medial of the medial/lateral midline; and

a hinge post having an longitudinal axis, the hinge post received in a recess in the baseplate including in the keel.

2. The prosthesis assembly of claim 1, wherein the longitudinal axis of the hinge post is medially biased toward the medial compartment so as to be spaced a second distance medial of the medial/lateral midline.

3. The prosthesis assembly of claim 2, wherein the first distance is substantially the same as the second distance.

4. The prosthesis assembly of claim 3, wherein the longitudinal axis of the hinge post and the longitudinal keel axis are substantially coaxially aligned for at least three different standard stock sizes of the baseplate.

5. The prosthesis assembly of any one of claims 1-4, wherein the longitudinal axis of the hinge post is offset laterally from the longitudinal keel axis for at least three different standard stock sizes of the baseplate.

6. The prosthesis assembly of claim 5, wherein a distance of the offset is between 0.25 mm and 2.5 mm, inclusive.

7. The prosthesis assembly of any one of claims 1-6, wherein the longitudinal axis of the hinge post is offset medially from the medial/lateral midline for all different standard stock sizes of the baseplate.

8. The prosthesis assembly of any one of claims 1-7, further comprising a femoral component connected to the hinge post, wherein a medial-lateral midline of the femoral component is offset medially with respect to the medial/lateral midline of the baseplate.

9. The prosthesis assembly of any one of claims 1-8, wherein a minimum wall thickness of the keel at a lateral side portion is at least 1.25 mm.

10. The prosthesis assembly of any one of claim 1-9, wherein a proximal end portion of the hinge post has a jog portion that extends medially from the proximal end portion toward a distal end portion of the hinge post.

11. The prosthesis assembly of any one of claims 1-10, wherein the hinge post is rotatable relative to the baseplate about the longitudinal axis.

12. The prosthesis assembly of claim 1, wherein the hinge post is one of a plurality of hinge posts, wherein when at least one of the plurality of hinge posts is received in the recess, the longitudinal axis of the hinge post and the longitudinal keel axis are substantially coaxially aligned, and wherein for two or more of the plurality of hinge posts when each of these two of the plurality of hinge posts is received in the recess the longitudinal axis is offset laterally from the longitudinal keel axis.

13. The system of claim 12, wherein longitudinal keel axis is medially biased toward the medial compartment so as to be spaced a first distance medial of the medial/lateral midline.

14. The system of claim 1, wherein the longitudinal axis of the hinge post is medially biased toward the medial compartment so as to be spaced a second distance medial of the medial/lateral midline, and wherein at least one of:

a medial-lateral midline of the femoral component is offset medially with respect to the medial/lateral midline of the baseplate; or

a proximal end portion of the hinge post has a jog portion that extends medially from the proximal end portion toward a distal end portion of the hinge post.

FIG. 1

FIG. 1A

**FIG. 2**

FIG. 3

EP 4 389 080 A1

100, 144

FIG. 4

**Medialized Keel Offset Distance (mm) From Midline and Medialized Axis of Rotation/Articulation of Hinge Post from Midline (mm)**

Legend:
- —□— Axes Shared in Common
- —⊘— Medialized Keel Offset Distance (MM) From Midline
- - -○- - Medialized Axis of Rotation/Articulation of Hinge Post from Midline

X-axis: Size (0 to 8)
Y-axis: 0 to 3.5

**FIG. 4A**

17

**FIG. 5**

FIG. 5A

**FIG. 6**

**FIG. 7A**

Femoral Component Shifted Medially Relative to Baseplate

**FIG. 7B**

EP 4 389 080 A1

FIG. 8

**FIG. 9**

Europäisches
Patentamt

European
Patent Office

Office européen
des brevets

## EUROPEAN SEARCH REPORT

Application Number

EP 23 21 8009

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2013/131820 A1 (WENTORF MARY S S [US] ET AL) 23 May 2013 (2013-05-23) | 1-7,9,11 | INV. A61F2/38 |
| Y | * paragraphs [0009] - [0012], [0094] - | 8,14 | |
| A | [0122] * | 13 | ADD. |
| | * figures 8-9B, 14 * | | A61F2/30 |
| | ----- | | |
| X | US 2003/055509 A1 (MCCUE DIANA F [US] ET AL) 20 March 2003 (2003-03-20) | 1-4,9,11 | |
| A | * paragraphs [0027] - [0036] * | 13,14 | |
| | * figures 1-14 * | | |
| | ----- | | |
| X | FR 2 949 668 A1 (OHST MEDIZINTECHNIK AG ET ISO [DE] ET AL.) 11 March 2011 (2011-03-11) | 1-4,9,11 | |
| A | * pages 3-9 * | 13,14 | |
| | * figures 1-4 * | | |
| | ----- | | |
| X | US 2013/024001 A1 (WENTORF MARY S S [US] ET AL) 24 January 2013 (2013-01-24) | 1-4,9,11 | |
| A | * paragraphs [0109] - [0161] * | 13,14 | |
| | * figures 4A-11 * | | TECHNICAL FIELDS SEARCHED (IPC) |
| | ----- | | |
| X | US 2019/038417 A1 (YOKO TIM [US] ET AL) 7 February 2019 (2019-02-07) | 1-7,9-13 | A61F |
| A | * paragraphs [0019] - [0050] * | 14 | |
| | * figures 1-6 * | | |
| | ----- | | |
| Y | US 2018/028324 A1 (CLARY CHADD W [US] ET AL) 1 February 2018 (2018-02-01) | 8,14 | |
| | * paragraphs [0056] - [0080] * | | |
| | * figures 4-14 * | | |
| | ----- | | |

The present search report has been drawn up for all claims

| Place of search | Date of completion of the search | Examiner |
|---|---|---|
| Munich | 19 April 2024 | Felkel, Bernd |

EPO FORM 1503 03.82 (P04C01)

## ANNEX TO THE EUROPEAN SEARCH REPORT
## ON EUROPEAN PATENT APPLICATION NO.

EP 23 21 8009

This annex lists the patent family members relating to the patent documents cited in the above-mentioned European search report.
The members are as contained in the European Patent Office EDP file on
The European Patent Office is in no way liable for these particulars which are merely given for the purpose of information.

19-04-2024

| Patent document cited in search report | | Publication date | Patent family member(s) | | Publication date |
|---|---|---|---|---|---|
| US 2013131820 | A1 | 23-05-2013 | AU | 2012341026 A1 | 10-07-2014 |
| | | | CA | 2856571 A1 | 30-05-2013 |
| | | | CN | 104093380 A | 08-10-2014 |
| | | | CN | 106214292 A | 14-12-2016 |
| | | | EP | 2782525 A1 | 01-10-2014 |
| | | | EP | 3037068 A1 | 29-06-2016 |
| | | | EP | 3241527 A1 | 08-11-2017 |
| | | | ES | 2585838 T3 | 10-10-2016 |
| | | | JP | 5824163 B2 | 25-11-2015 |
| | | | JP | 6077082 B2 | 08-02-2017 |
| | | | JP | 2015504333 A | 12-02-2015 |
| | | | JP | 2016028729 A | 03-03-2016 |
| | | | US | 2013131820 A1 | 23-05-2013 |
| | | | US | 2014249641 A1 | 04-09-2014 |
| | | | US | 2016158019 A1 | 09-06-2016 |
| | | | US | 2017266011 A1 | 21-09-2017 |
| | | | WO | 2013077919 A1 | 30-05-2013 |
| US 2003055509 | A1 | 20-03-2003 | DE | 69918894 T2 | 11-08-2005 |
| | | | EP | 0956836 A1 | 17-11-1999 |
| | | | JP | 4115625 B2 | 09-07-2008 |
| | | | JP | 2000000255 A | 07-01-2000 |
| | | | US | 6506216 B1 | 14-01-2003 |
| | | | US | 2003055509 A1 | 20-03-2003 |
| FR 2949668 | A1 | 11-03-2011 | NONE | | |
| US 2013024001 | A1 | 24-01-2013 | AU | 2012368262 A1 | 18-09-2014 |
| | | | AU | 2017235987 A1 | 19-10-2017 |
| | | | CA | 2863375 A1 | 08-08-2013 |
| | | | CN | 104203160 A | 10-12-2014 |
| | | | EP | 2809273 A1 | 10-12-2014 |
| | | | ES | 2869958 T3 | 26-10-2021 |
| | | | JP | 6138160 B2 | 31-05-2017 |
| | | | JP | 2015504759 A | 16-02-2015 |
| | | | KR | 20140133836 A | 20-11-2014 |
| | | | US | 2013024001 A1 | 24-01-2013 |
| | | | US | 2016287397 A1 | 06-10-2016 |
| | | | US | 2018000602 A1 | 04-01-2018 |
| | | | WO | 2013115849 A1 | 08-08-2013 |
| US 2019038417 | A1 | 07-02-2019 | NONE | | |
| US 2018028324 | A1 | 01-02-2018 | AU | 2017204218 A1 | 15-02-2018 |
| | | | CN | 107661161 A | 06-02-2018 |
| | | | EP | 3275406 A1 | 31-01-2018 |

EPO FORM P0459

For more details about this annex : see Official Journal of the European Patent Office, No. 12/82

page 1 of 2

## ANNEX TO THE EUROPEAN SEARCH REPORT
## ON EUROPEAN PATENT APPLICATION NO.

EP 23 21 8009

This annex lists the patent family members relating to the patent documents cited in the above-mentioned European search report.
The members are as contained in the European Patent Office EDP file on
The European Patent Office is in no way liable for these particulars which are merely given for the purpose of information.

19-04-2024

| Patent document cited in search report | Publication date | Patent family member(s) | | Publication date |
|---|---|---|---|---|
| | | EP | 3556324 A1 | 23-10-2019 |
| | | JP | 2018015571 A | 01-02-2018 |
| | | US | 2018028324 A1 | 01-02-2018 |
| | | US | 2019142595 A1 | 16-05-2019 |
| | | US | 2020030107 A1 | 30-01-2020 |
| | | US | 2021228367 A1 | 29-07-2021 |

EPO FORM P0459

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 8758444 B2
- US 9381090 B2

- US 5282861 A, Kaplan